(19) **Europäisches Patentamt**

**European Patent Office**

**Office européen des brevets**

(11) **EP 1 024 681 A2**

(12) **EUROPÄISCHE PATENTANMELDUNG**

(43) Veröffentlichungstag:
02.08.2000 Patentblatt 2000/31

(51) Int. Cl.⁷: **H05G 1/26**

(21) Anmeldenummer: **00200226.9**

(22) Anmeldetag: **20.01.2000**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **30.01.1999 DE 19903749**

(71) Anmelder:
• **Philips Corporate Intellectual Property GmbH**
**52064 Aachen (DE)**
Benannte Vertragsstaaten:
**DE**

• **Koninklijke Philips Electronics N.V.**
**5621 BA  Eindhoven (NL)**
Benannte Vertragsstaaten:
**FR GB NL**

(72) Erfinder: **Zimmermann, Robert**
**52064 Aachen (DE)**

(74) Vertreter:
**Volmer, Georg, Dipl.-Ing. et al**
**Philips Corporate Intellectual Property GmbH,**
**Habsburgerallee 11**
**52064 Aachen (DE)**

(54) **Röntgen-Diagnostikeinrichtung mit Mitteln zur Bestimmung der Dosis**

(57)     Die Erfindung betrifft eine Röntgen-Diagnostikeinrichtung, bei der die einem Patienten applizierte Dosis ermittelt wird und bei der die für die Strahlenbelastung relevante Effektivdosis aus dieser Dosis sowie aus einem Wichrungsfaktor berechnet wird, der in Abhängigkeit von der jeweils untersuchten anatomischen Region aufgerufen wird.

**EP 1 024 681 A2**

**Beschreibung**

Die Erfindung betrifft eine Röntgen-Diagnostikeinrichtung mir einem Röntgengenerator zur Speisung eines Röntgenstrahlers und mit Mitteln zur Bestimmung der bei einer Röntgenuntersuchung eines Patienten applizierten Dosis.

Eine solche Röntgen-Diagnostikeinrichtung ist aus der DE-OS 2 124 035 (Seite 2, 2. Abs.) bekannt. Die Dosis wird dabei mittels einer Ionisationskammer gemessen, die an einer mit dem Röntgenstrahler verbundenen Ionisationskammer befestigt ist. Diese Ionisationskammer ist so großflächig ausgebildet, daß sie auch bei der maximalen, mit der Primärstrahlenblende einstellbaren Strahlenfeldgröße die gesamte aus der Primärstrahlenblende austretende Röntgenstrahlung erfaßt. Das Ausgangssignal der Ionisationskammer entspricht somit dem ¥ in µGy*m$^2$ gemessenen ¥ Dosisflächenprodukt.

Das Dosisflächenprodukt, ist eine rein physikalische Größe, das dem Flächenintegral des Kerma (kinetic energy released in matter) in Luft entspricht. Diese physikalische Größe sagt nur bedingt etwas über die Strahlenbelastung des untersuchten Patienten aus bzw. über die Effektivdosis, die die unterschiedlichen Strahlenrisiken der einzelnen Organe oder Gewebe bezüglich stochastischer Strahlenwirkungen berücksichtigt. Bei gleichem applizierten Dosisflächenprodukt wird beispielsweise die Blase eines Patienten erheblich stärker durch die Röntgenstrahlung geschädigt als sein Schädel.

Aufgabe der vorliegenden Erfindung ist es daher, eine Röntgen-Diagnostikeinrichtung der eingangs genannten Art so auszugestalten, daß die Strahlenbelastung eines Patienten bei einer Röntgenuntersuchung genauer angegeben werden kann. Diese Aufgabe wird erfindungsgemäß dadurch gelöst, daß eine Speicheranordnung vorgesehen ist, in der für eine Anzahl von Organen je ein Satz von Aufnahme- und/oder Durchleuchtungs-parametern gespeichert ist, daß jeder Satz neben Aufnahme- bzw. Durchleuchtungsparametern für den Röntgengenerator einen der biologischen Wirksamkeit der Strahlung für das jeweilige Organ entsprechenden Wichtungsfaktor enthält und daß eine Recheneinheit zur Berechnung der Effektivdosis aus der Dosis und dem Wichtungsfaktor vorgesehen ist.

Die Verwendung eines Speichers, in dem für eine Anzahl von Organen je ein Satz von Aufnahme- bzw. Durchleuchtungsparametern gespeichert ist, ist bei Röntgengeneratoren heutzutage üblich und wird als APR-Einstellung bezeichnet (APR=anatomicaly programmed radiography). Bei einer Röntgenaufnahme gibt der Benutzer das zu untersuchende Organ - bzw. die anatomische Region - vor (z.B. "Lunge p.a."), wobei in dem Speicher die für eine Röntgenaufnahme dieses Organs optimalen Aufnahmeparameter (u.a. die Spannung an dem Röntgenstrahler) aufgerufen und automatisch eingestellt werden.

Die Erfindung basiert nun auf der Überlegung, daß man jedem Organ einen Wichtungsfaktor zuordnen kann, der ein Maß für die biologische Wirksamkeit der Dosis bei einer Röntgenuntersuchung des betreffenden Organs ist. Durch Multiplikation dieses Wichtungsfaktors mir der während der Röntgenuntersuchung ermittelten Dosis (d.h. dem Kerma in Luft) läßt sich somit die Effektivdosis berechnen, die ein Maß für die biologische Wirksamkeit der Röntgenstrahlung in dem Patienten darstellt.

Die Erfindung ist nicht nur bei Röntgengeräten anwendbar, mit denen man lediglich Röntgenaufnahmen anfertigen kann ¥ wie den sogenannten Bucky-Einrichtungen ¥ sondern auch bei Röntgeneinrichtungen, mit denen sowohl ein Aufnahmeberrieb als auch ein Durchleuchtungsbetrieb möglich ist. Bei modernen Geräten dieser Art können auch die Durchleuchtungsparameter für verschiedene Organe bzw. anatomische Regionen gespeichert sein, wobei bei einem Übergang zu einer Röntgenaufnahme die Aufnahmeparameter entweder aus den Durchleuchtungsparametern abgeleitet werden oder aus einem gesonderten Speicher mit den Aufnahmeparametern für das betreffende Organ entnommen werden.

Die Dosis bzw. das Dosisflächenprodukt können mit einer geeigneten Meßkammer erfaßt werden. Die Weiterbildung nach Anspruch 2 kommt ohne eine solche Meßkammer aus, wobei die Dosis einerseits aus den Aufnahme- bzw. Durchleuchtungsparametern abgeleitet wird (Spannung am Röntgenstrahler bzw. zeitliches Integral des Stromes durch den Röntgenstrahler, Filterung usw.) und andererseits aus geometrischen Parametern der Röntgenuntersuchung (z.B. der Öffnung des Röntgenstrahlenbündels) abgeleitet werden.

Eine bevorzugte Ausgestaltung der Mittel zum Erfassen der geometrischen Parameter ist in Anspruch 3 angegeben.

Die Erfindung wird nachstehend anhand einer Zeichnung erläutert, die - rein schematisch ¥ eine erfindungsgemäße Röntgeneinrichtung darstellt.

Ein Röntgengenerator 1 liefert die Hochspannung für einen Röntgenstrahler 2 sowie den Strom durch den Röntgenstrahler. In einer am Röntgenstrahler 2 befestigten Primärstrahlenblende 3 befindet sich ein erstes Kollimatorpaar 31 mit zur Zeichenebene senkrechten Kollimatorkanten, das die Öffnung des von dem Röntgenstrahler ausgeblendeten Strahlenbündels 4 in der Zeichenebene bestimmt. Ein zweites, nicht näher dargestelltes Kollimator-paar mit zur Zeichenebene parallelen Kollimatorkanten bestimmt die Öffnung des Strahlenbündels 4 in der zur Zeichenebene senkrechten Richtung. Das EP einen Patienten 5, der sich auf einem Patientenlagerungstisch 6 befindet. Das als Folge EP der Durchstrahlung entstehende Röntgenbild wird von einem geeigneten Bildwandler EP7 aufgezeichnet.

Die von dem Röntgengenerator erzeugte Hochspannung für und der Strom durch den Röntgenstrahler 2 sowie der zeitliche Verlauf dieser Größen werden von einer Steuereinheit 8 vorgegeben, beispielsweise einem Mikroprozessor, der mit einem Speicher 9 zusammenwirkt. Die Steuereinheit 8 ist mit einer Anzeigeeinheit bzw. einem Monitor 10 gekoppelt und außerdem mit einer Eingabeeinheit 11 (Tastatur, Maus oder Trackball), mit der ein Benutzer den Verlauf der nachfolgenden Röntgenuntersuchung bestimmen kann und mit der er insbesondere vorgehen kann, welches Organ bzw. welche anatomische Region bei einer nachfolgenden Röntgenaufnahme untersucht werden soll.

Die Effektivdosis $D_{eff}$ berechnet sich nach der Formel:

$$D_{eff} = c \cdot \gamma \cdot Q \cdot A \cdot w$$

Dabei ist c eine Konstante und $\gamma$ ein Faktor, der von der Hochspannung U an der Röntgenröhre sowie dem im Strahlengang wirksamen, in der der Primärstrahlenblende 3 befindlichen Filter 35 abhängt. Q entspricht dem zeitlichen Integral des Stromes durch die Röntgenröhre (in der Regel ist das der Röhrenstrom multipliziert mit der Dauer einer Röntgenaufnahme), und A stellt die Öffnung des Strahlenbündels bzw. die durch die Kollimatorpaare 31 begrenzte Querschnittsfläche des Strahlenbündels dar. Das Produkt $c \cdot \gamma \cdot Q \cdot A \cdot$ entsprichtr der Dosis D. w ist ein Wichtungsfaktor, der die biologische Wirksamkeit der Röntgenstrahlung in dem jeweils untersuchten anatomischen Bereich beschreibt.

Bei einer Röntgenaufnahme gibt der Untersucher an der Eingabeeinheit 11 den zu untersuchenden Bereich, wie z.B. "Lunge p.a." vor. Die Steuereinheit 8 ruft dann die für eine Aufnahme dieser Region optimalen, in der Speicheranordnung 9 gespeicherten Aufnahmeparameter, nämlich die Röhrenspannung U und das dem Faktor Q entsprechende mAs-Produkt, sowie einen Wichtungsfaktor w auf, der der betreffenden anatomischen Region zugeordnet ist. Aus der auf diese Weise aufgerufenen Röhrenspannung und der im Strahlengang wirksamen Filterung, die durch das Signal F der Steuereinheit 8 übermittelt wird, berechnet diese den Faktor $\gamma$. Die Querschnittsfläche A des Strahlenbündels wird ihr von einer Geberanordnung 33 übermittelt, die beispielsweise mit den Kollimatorpaaren in der Primärstrahlenblende gekoppelte Potentiometer umfassen kann. Aus den vorgegebenen bzw. in der Steuereinheit 8 ermittelten Parametern kann diese dann die bei der Aufnahme der anatomischen Region verabfolgte Effektivdosis berechnen.

In analoger Weise kann die Effektivdosis $D_{eff}$ auch bei einer Röntgendurchleuchtung ermittelt werden, wobei in Abhängigkeit von der vorgegebenen anatomischen Region der Wichtungsfaktor w und die Röhrenspannung U aus der Speicheranordnung 9 aufgerufen wird. Die Größe Q wird dann von der Steuereinheit durch Bildung des zeitlichen Integrals über den durch die Röntgenröhre während der Durchleuchtung fließenden Röhrenstrom gebildet.
Wenn die Kollimatorpaare 31 weit geöffnet sind, können Teile von anderen Organen in den Strahlengang gelangen, für die die Röntgenstrahlung eine andere biologische Wirksamkeit hat. Dies kann zur Folge haben, daß bei einer Aufnahme mit einem großen Strahlungsfeld ein anderer Wichtungsfaktor angesetzt werden muß, als bei einer Aufnahme mit einem Meinen Strahlungsfeld. Dies läßt sich dadurch berücksichtigen, daß der Wichtungsfaktor w auch von der Querschnittsfläche A der Kollimatoranordnung abhängig gemacht werden muß. Dies kann beispielsweise dadurch geschehen, daß für eine anatomische Region mehrere Wichtungsfaktoren in der Speicheranordnung 9 gespeichert sind von denen eine in Abhängigkeit von dem jeweiligen Wert A ermittelt wird und der Berechnung der Effektivdosis zugrundegelegt wird.

Wie bereits erläutert, kann man die Dosis D statt durch eine Berechnung auch durch eine Messung ermitteln. Die Effektivdosis ergibt sich dann durch Multiplikation des Meßwertes mit einem Wichrungsfaktor w.

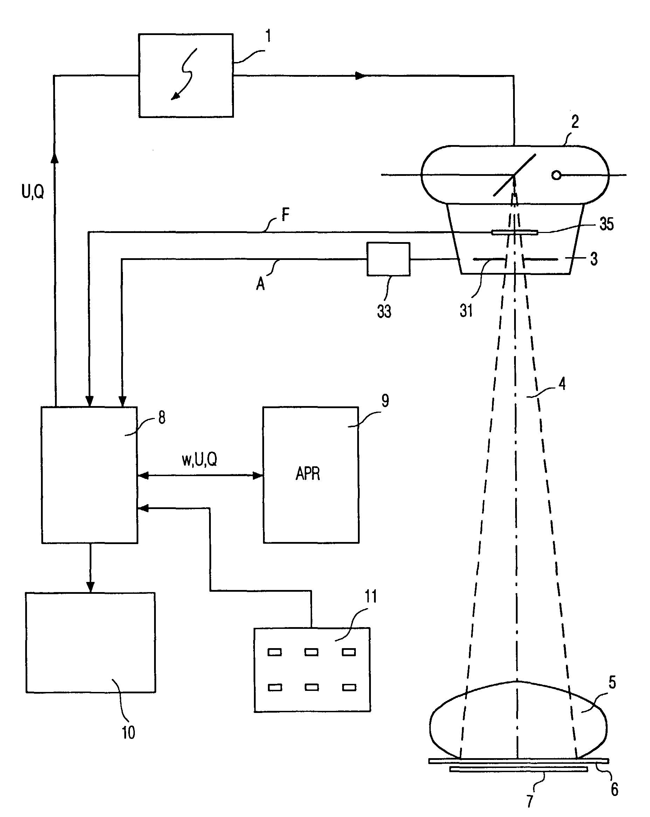

**Patentansprüche**

1. Röntgen-Diagnostikeinrichtung mit einem Röntgengenerator (1) zur Speisung eines Röntgenstrahlen (2) und mit Mitteln zur Bestimmung der bei einer Röntgenuntersuchung eines Patienten applizierten Dosis
dadurch gekennzeichnet, daß eine Speicheranordnung (9) vorgesehen in der für eine Anzahl von Organen je ein Satz von Aufnahme- und/oder Durchleuchtungsparametern gespeichert ist, daß jeder Satz neben Aufnahme- bzw Durchleuchtungsparametern (U,Q) für den Röntgengenerator einen der biologischen Wirksamkeit der Strahlung für das jeweilige Organ entsprechenden Wichtungsfaktor (w) enthält und daß eine Recheneinheit zur Berechnung der Effektivdosis ($D_{eff}$) aus der Dosis und dem Wichtungsfaktor vorgesehen ist.

2. Röntgen-Diagnostikeinrichtung nach Anspruch 1 dadurch gekennzeichnet, daß Mittel (33) zum Erfassen der geometrischen Parameter der Röntgenuntersuchung und Mittel (8) zum Berechnen der Dosis aus den geometrischen Parametern und den Aufnahme bzw Durchleuchtungsparametern vorgesehen sind.

3. Röntgen-Diagnostikeinrichtung nach Anspruch 1 dadurch gekennzeichnet, daß die Mittel zum Erfassen der geometrischen Parameter eine Meßeinrichtung (33) zur Messung der Öffnung einer mir dem Röntgenstrahler verbundenen Primärstrahlenblende umfassen.